# EUROPÄISCHE PATENTANMELDUNG

(11) **EP 2 042 489 A1**
(43) Veröffentlichungstag der Anmeldung: **01.04.2009**
(21) Anmeldenummer: 07117221.7
(22) Anmeldetag: 26.09.2007
(51) Int. Cl.: C07D 213/70

(54) **Beseitigen und Verhindern von Verfärbungen Pyrithion enthaltender Stoffe**

(71) Anmelder: Straetmans high TAC GmbH, 22143 Hamburg (DE)
(72) Erfinder: Straetmans, Udo, Dr., 22143 Hamburg (DE); Straetmans, Felix, 22143 Hamburg (DE)
(74) Vertreter: Raffay & Fleck

(57) **Zusammenfassung**

Die Erfindung beschäftigt sich mit Pyrithionen, die bekannte antimikrobielle Produkte darstellen, die in einer Vielzahl von Anwendungen zur Konservierung nützlich verwendet werden. Die Pyrithione und ihre Salze besitzen jedoch die Eigenschaft mit Schwermetallen intensiv gefärbte Verbindungen einzugehen, die nicht akzeptabel sind und auch zu erheblichen technischen Problemen führen. Hier schafft die Erfindung Abhilfe durch die Verwendung eines oder mehrerer Polyamine und/oder deren Derivate zum Entfärben und/oder zum Verhindern von durch Schwermetallionen, insbesondere Eisen-III-Ionen hervorgerufener Verfärbungen von Pyrithion und/oder Salze desselben enthaltenden Stoffen.

## Beschreibung

### Technisches Gebiet

Diese Erfindung betrifft die Verwendung einer bestimmten Chemikalie sowie ein Verfahren zum Entfärben und/oder zum Verhindern von Verfärbungen von Pyrithion enthaltenden technischen Stoffen, die eine antimikrobielle Wirkung besitzen. Des weiteren ist die Erfindung auf den technischen Stoff selbst und ein Additiv gerichtet.

### Stand der Technik

Pyrithione sind bekannte antimikrobielle Stoffe, die in einer Vielzahl von Anwendungen zur Konservierung nützlich verwendet werden. Natrium-Pyrithion (CAS 3811-73-2) ist ein wasserlösliches Pyrithion-Salz mit sehr guten antimikrobiellen Eigenschaften und wird z.B. als Konservierungsmittel (Biozid) in technischen Prozessflüssigkeiten und Hilfsmitteln wie z.B. Metallbearbeitungsflüssigkeiten, Schmierstoffen, Bindemitteln, Klebstoffen, Dichtungsmitteln, Lederhilfsmitteln, Papierbeschichtungsmitteln und sogar kosmetischen Präparaten verwendet. Die Herstellung von Natrium-Pyrithion wurde erstmals im US-Patent No. 3.159.640 beschrieben.

Leider besitzt Natrium-Pyrithion und weitere Salze, z.B. ZinkPyrithion die Eigenschaft mit Schwermetallen, vor allem Eisenionen, intensiv gefärbte (dunkelgrün, blau, schwarz) Verbindungen einzugehen. Selbst wenn diese nur in Spuren vorhanden sind, z.B. als Katalysatorreste in Chemikalien oder natürliche Begleitstoffe (Eisen) von Additiven für Farben und Lacke (etc. oben genannte). Diese Verfärbungen sind nicht nur aus ästhetischen Gründen nicht akzeptabel, sondern führen auch zu massiven technischen Problemen wie z.B. Wirkungsverlust.

Ein Ölkonzentrat unterliegt einer bestimmten Spezifikation bei der Farbe, z.B. braun. Ein schwarzes Produkt ist nicht verkäuflich. Metallbearbeitungsflüssigkeiten werden normalerweise als weiße Emulsionen oder schwach gelblich gefärbte Lösungen verwendet. Dunkelblaue bis schwarze Flüssigkeiten, die Werkstücke und Maschinen schwarz einfärben, führen zu eklatanten finanziellen Verlusten. In der Vergangenheit hat es Versuche zur Lösung der Verfärbung gegeben, die alle zu mehr oder weniger starken Nebeneffekten führen. In diesen Fällen wird versucht die Schwermetalle, besonders Eisenionen, durch andere Metallionen zu verdrängen bzw. durch Komplexbildung zu beseitigen. (Die Reaktion von Fe-III-Ionen mit Pyrithion führt zu einem schwarzen Niederschlag und wird so dem System entzogen. Damit verbunden ist ein Verlust an biozider Wirkung.) In Metallbearbeitungsflüssigkeiten führt die Verwendung von Komplexbildnern zwar zur Entfärbung von Fe-Pyrithion, vermindert allerdings auch die Wasserhärte und führt fast immer zur unerwünschten Schaumbildung der Flüssigkeit und Schaum kann weder schmieren noch kühlen. Eine weitere Möglichkeit ist die Verwendung von Salzen, die mit Pyrithion zu ungefärbten Komplexsalzen führen, z.B. Zn-Salzen (US-Patent No. 4.161.526). Auch diese Verfahrensweise führt zwar zu einer Entfärbung, allerdings auch zu einer Verminderung der bioziden Wirkung des Pyrithions und zu unerwünschten Folgeschäden.

### Darstellung der Erfindung

Der vorliegenden Erfindung liegt die Aufgabe zugrunde, Verfärbungen von pyrithionhaltigen, technischen Produkten wie z.B. Kühlschmierstoffe (KSS), Farben und Lacke, Klebstoffe, Dichtungsmittel, Lederhilfsmittel, Papierbeschichtungsmittel und Kosmetika ausschließen und ggf. Entfärbungen erzielen zu können, ohne dass die biozide Wirkung des Pyrithions vermindert wird. Auch sollten die Verwendungspotentiale erhöht, die Anwendung erleichtert und preiswerter gestaltet, die Wirksamkeit erhöht, bzw. gewährleistet und sichergestellt und Regressansprüche vermieden werden.

Diese Aufgabe wird durch die Verwendung eines oder mehrerer Polyamine und/oder deren Derivate zum Entfärben und/oder zum Verhindern von durch Schwermetallionen, insbesondere Eisen-III-Ionen hervorgerufener Verfärbungen von Pyrithion und/oder Salze desselben enthaltenden Stoffes gelöst. Weitere Lösungsvorschläge geben die Ansprüche 6, 7 und 9.

Es wurde auch für Experten völlig überraschend gefunden, dass Polyamine und deren Derivate sowohl Verfärbungen z.B. in Ölkonzentraten als auch in Metallbearbeitungsflüssigkeiten in Gegenwart von Schwermetallen, insbesondere Fe-III-Ionen, spontan entfärben und einer weiteren Verfärbung auch langfristig entgegenwirken. Solche Ölkonzentrate sind allgemein wie folgt aufgebaut und können bei Raumtemperatur in der angegebenen Reihenfolge gemischt werden:

- bis 70% Basisöl (naphthalbasisch, parafinbasisch, z.B. Nynäs T22)

- 20% - 40% Emulgatorsystem / Korrosionsschutz (Petrolsulfonate, Kalium- oder Amin-Seifen, Nichtionische

Tenside, C12 - C18 Ethoxylate)

- bis 40% Lösungsvermittler / Wasser

- 0,01 -5,0% insbesondere 0,1% - 3% Natriumpyrithion (Biozid)

- 0,01 % - 20% Polyamine und deren Derivate

Diese allgemeine Rezeptur der vorliegenden Erfindung zeigt die vorteilhafte Kombination des antimikrobiellen Stoffes mit Polyaminen und deren Derivaten. Die notwendige Dosierung der Polyamine beträgt das 1/5-10-fache der Konzentration wie Natriumpyrithion und liegt insbesondere in der ungefähr gleichen Konzentration. Die erfindungsgemäß beschriebenen Polyamine sind nicht nur zur Verhinderung von Verfärbungen geeignet, sondern können auch Amine ersetzen, die für die in der Rezeptur angegebenen Aminseifen aufgewandt werden müssen. Die eingesetzten Polyamine können ebenfalls zum Emulgieren wie zum Korrosionsschutz beitragen. Die Dosierung der Polyamine kann also vorteilhaft im Emulgatorsystem/Korrosionsschutz eingesetzt werden. Die Verwendung der erfindungsgemäß beschriebenen Polyamine beschränkt sich nicht nur auf hoch ölhaltige Kühlschmierstoffe, sondern ist universell über halbsynthetische Produkte bis zu klar wasserlöslichen Vollsythesen geeignet.

### Weg(e) zur Ausführung der Erfindung

Folgende Beispiele der Polyamine und deren Derivate sollen die Erfindung beschreiben, aber in keiner Weise beschränken:

Beispiel A:

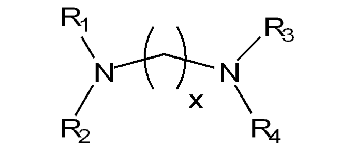

- x ∈ N, linear/ verzweigt gesättigt, linear/ verzweigt ungesättigt

- R₁,R₂,R₃,R₄ = H, gesättigt/ungesättigt verzweigt/linear Alkyl C1-C10, Cycloalkyl, Ethoxylate, Propoxylate, Alkylaryl, Heterocyclen.

Ein vollsynthetischer KSS (klare, farblose Flüssigkeit) bzw. dessen 5 %ige wässrige Lösung enthält 0,1 % eines Konservierungsmittels enthaltend 10 % Natrium-Pyrithion. In der klaren Lösung befinden sich also 100 ppm Natrium-Pyrithion. Dieser Lösung werden 0,01 g einer 5 %igen NH₄ Fe(III) (SO₄)₂ - Lösung zugetropft. Es entsteht spontan eine intensiv tiefblau gefärbte Flüssigkeit. Nach Zugabe von 0,05 g Diethylaminoethylamin wird die Lösung spontan entfärbt.

Von den oben allgemein dargestellten Produkten eignen sich besonders:
- X = 2; R₃ = R₄ = H; R₁ = R₂ = C₂H₅ Diethylaminoethylamin CAS 100-36-7
- X = 3; R₃ = R₄ = H; R₁ = R₂ = Diethylaminopropylamin CAS 104-78-9
- X = 3; R₃ = R₄ = H; R₁ = R₂ = N-Piperidyl /N-aminopropylpiperidin
- X = 3; R₃ = R₄ = H; R₁ = R₂ = Morpholyl /N-Aminopropylmorpholin CAS 123-00-2

Beispiel B:

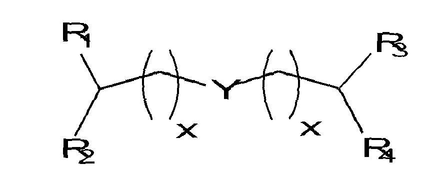

- Y = O, NH, NR₁, R₁-C=C-R₂, Cycloalkyl, EO, PO, CH₂

- x ∈ N, linear/ verzweigt gesättigt, linear/ verzweigt ungesättigt

- R₁,R₂,R₃,R₄ = H, gesättigt/ungesättigt verzweigt/linear Alkyl C1-C10, Cycloalkyl, Ethoxylate, Propoxylate, Alkylaryl, Heterocyclen.

Beispiel C:

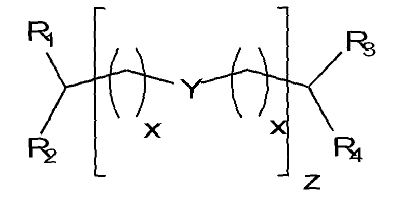

- Y = O, NH, NR₁, R₁-C=C-R₂, Cycloalkyl, EO, PO, CH₂

- x ∈ N, linear/ verzweigt gesättigt, linear/ verzweigt ungesättigt

- R₁,R₂,R₃,R₄ = H, gesättigt/ungesättigt verzweigt/linear Alkyl C1-C10, Cycloalkyl, Ethoxylate, Propoxylate, Alkylaryl, Heterocyclen.

- z ∈ N

In einer klaren Lösung eines Kühlschmierstoffes befinden sich 100 ppm Natrium-Pyrithion. Dieser Mischung werden 0,01 g einer 5 %igen NH₄ Fe(III) (SO₄)₂ - Lösung zugetropft. Es entsteht ummittelbar eine tiefblaue Lösung. Nach Zugabe von 0,05 g Diethylentriamin erhält man sofort eine wasserklare Flüssigkeit. Von den allgemein dargestellten Produkten eignen sich besonders:
1. Diethylentriamin CAS 111-40-0
2. Bis-(3-aminopropyl)Ether CAS 2579-20-6
3. 1,3-Bis(aminomethyl)Cyclohexan CAS 2579-20-6
4. Polyetheramin CAS 39423-51-3

## Patentansprüche

1. Verwendung eines oder mehrerer Polyamine und/oder deren Derivate zum Entfärben und/oder zum Verhindern von durch Schwermetallionen, insbesondere Eisen-III-Ionen, hervorgerufener Verfärbungen von Pyrithion und/oder Salze desselben enthaltenden Stoffen.

2. Verwendung nach Anspruch 1, **dadurch gekennzeichnet, dass** die Konzentration des oder der Polyamine und/oder deren Derivate 0,2 bis 10-fache der Konzentration des Pyrithion und/oder des Salzes desselben beträgt.

3. Verwendung nach Anspruch 1 und 2, **dadurch gekennzeichnet, dass** die Stoffe Ölkonzentrate, insbesondere Kühlschmierstoffkonzentrate, Farben und Lacke, Klebstoffe, Dichtungsmittel, Lederhilfsmittel, Papierbeschichtungsmittel und Kosmetika darstellen.

4. Verwendung nach Anspruch 1 bis 3, **dadurch gekennzeichnet, dass** die Kühlschmierstoffkonzentrate einen Basisölanteil bis 70 Gew.%, einen Emulgator von 20 bis 40 Gew.%, einen Lösungsvermittler bis 40 Gew.%, sowie 0,01 bis 5,0 Gew.% Natriumpyrithion und Polyamine mit einem Anteil von 0,01 bis 20 Gew.%, jeweils bezogen auf die Gesamtmenge, enthalten.

5. Verwendung nach einem der Ansprüche 1 bis 4, **dadurch gekennzeichnet, dass** die Polyamine folgende Formel besitzen: 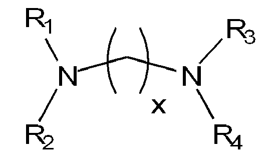 oder 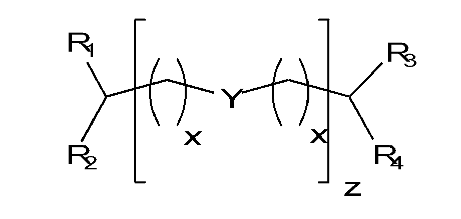 worin
- x ∈ N, linear/ verzweigt gesättigt, linear/ verzweigt ungesättigt
- R₁,R₂,R₃,R₄ = H, gesättigt/ungesättigt verzweigt/linear Alkyl C1-C10, Cycloalkyl, Ethoxylate, Propoxylate, Alkylaryl, Heterocyclen
- Y = O, NH, NR₁, R₁-C=C-R₂, Cycloalkyl, EO, PO, CH₂ oder nicht vorhanden
- z ∈ N

6. Verfahren zum Entfärben und/oder zum Verhindern von durch Schwermetallionen, insbesondere Eisen-III-Ionen, hervorgerufener Verfärbungen von Pyrithion und/oder Salze desselben enthaltenden Stoffen, insbesondere Kühlschmierstoffkonzentrate, Farben und Lacke, Klebstoffe, Dichtungsmittel, Lederhilfsmittel, Papierbeschichtungsmittel und Kosmetika, **gekennzeichnet durch** die Zugabe oder das Vorsehen eines Anteils von einem oder mehreren Polyaminen und/oder deren Derivaten.

7. Stoff, insbesondere Kühlschmierstoffkonzentrate, Farben und Lacke, Klebstoffe, Dichtungsmittel, Lederhilfsmittel, Papierbeschichtungsmittel und Kosmetika, Pyrithion und/oder Salze desselben enthaltend **gekennzeichnet durch** einen Anteil von einem oder mehreren Polyamin(en) und/oder deren Derivaten.

8. Stoff und deren wässrige Verdüngungen nach Anspruch 4, **gekennzeichnet durch** weitere Konservierungsmittel (Biozide) enthalten wie z.B. Hexahydrotriazine (HHT), Methylenbisoxazolidine (MBO), Methylenbismorpholine (MBM), DMDM-Hydantoine,(DMDMHy), Tetrahydro-1,3,4,6-tetrakis (hydroxymethyl)imidazo(4,5-d)imidazole-2,5(1 H,3H)-dion, Benzisothiazolinon (BIT) ,o-Phenylphenol (OPP), Jodpropinylbutylcarbamate (IPBC) und Mischungen davon.

9. Additiv, bestehend aus einem oder mehreren Polyamin(en) und/oder deren Derivaten und Pyrithion und/oder Salze desselben zur Verwendung in Stoffen, insbesondere Kühlschmierstoffkonzentrate, Farben und Lacke, Klebstoffe, Dichtungsmittel, Lederhilfsmittel, Papierbeschichtungsmittel und Kosmetika.

10. Additiv nach Anspruch 9, **dadurch gekennzeichnet, dass** die Konzentration des oder der Polyamine und/oder deren Derivate 0,2 bis 10-fache der Konzentration des Pyrithion und/oder des Salzes desselben beträgt.
